# EUROPEAN PATENT APPLICATION

(11) **EP 1 967 202 A1**
(43) Date of publication of application: **10.09.2008**
(21) Application number: 07103483.9
(22) Date of filing: 05.03.2007
(51) Int. Cl.: A61K 38/09, A61P 13/02, A61P 13/10, A61P 13/00

(54) **Use of LHRH Antagonists for the Treatment of Lower Urinary Tract Symptoms, in particular Overactive Bladder and/or Detrusor Overactivity**

(71) Applicant: AEterna Zentaris GmbH, 60314 Frankfurt (DE)
(72) Inventor: Engel, Jürgen, 63755, Alzenau (DE); Bauer, Oliver, 60487, Frankfurt (DE)

(57) **Abstract**

The present invention provides at least one LHRH antagonist for use in the preparation of a medicament for the treatment or prophylaxis of at least one lower urinary tract symptom in mammals, wherein the at least one lower urinary tract symptom is selected from the group consisting of: "urinary incontinence, urge incontinence, overactive bladder, idiopathic overactive bladder, neurogenic overactive bladder, detrusor overactivity, idiopathic detrusor overactivity, neurogenic detrusor overactivity" and wherein the at least one LHRH antagonist is to be administered in an intermediate dose, which does not cause chemical (hormonal) castration.

## Description

### Technical field

The invention relates to the use of LHRH antagonists for the treatment or prophylaxis of lower urinary tract symptom in mammals. Such lower urinary tract symptom comprise urinary incontinence, urge incontinence, overactive bladder, idiopathic overactive bladder, neurogenic overactive bladder, detrusor overactivity, idiopathic detrusor overactivity, neurogenic detrusor overactivity and benign prostatic hyperplasia (BPH). The invention further relates to the treatment or prophylaxis of above symptoms by administering LHRH antagonists in intermediate doses, which do not cause chemical (hormonal) castration.

### Prior art

Genitourinary problems, including neurogenic dysfunction, such as incontinence and urinary retention, impotence, prostatism, urinary tract infections (UTI), benign prostatic hyperplasia/hypertrophy (BPH) and prostate cancer, are common in the elderly, and most of the symptoms can be alleviated through pharmacological management (Atala A et al., Drugs & Aging 1991, 1(3): 176-193).

Overactive bladder (OAB) is a syndrome characterized by urgency with or without urge incontinence, usually with increased frequency and nocturia. Bladder overactivity can be myogenic or neurogenic in its origin or it can be idiopathic in nature. Myogenic etiology is characterized by impairment in smooth muscle function as a result of partial urethral obstruction. The increased smooth muscle signalling due to loss normal excitatory neural input leads to a state of unstable contractions or overactivity. OAB is also triggered by neurological effects resulting in dysregulation of reflexes to the bladder and urethra and leading to neurogenic detrusor overactivity.

In contrast, (urge) urinary incontinence is defined as an involuntary leakage accompanied by or immediately preceded by urgency (Tiwari A et al., Expert Opin. Investig. Drugs 2006, 15(9): 1017-1037).

In their review, Atala et al. (Atala A et al., Drugs & Aging 1991, 1(3): 176-193) mention the use of LHRH agonists, such as goserelin, leuprorelin, nafarelin and buserelin, to achieve castrate levels of androgens, for instance testosterone, in advanced prostatic carcinoma and BPH. The indication overactive bladder is not mentioned, neither are LHRH antagonists.

WO 02/36144 discloses the use of GnRH analogues for the treatment of side effects of ovarectomy or symptoms associated with reproductive senescence in female mammals, such as post-menopausal women and spayed bitches, in particular urinary incontinence, mood changes, hot flushes and skin/hair changes. The patent application mentions GnRH agonists and antagonists, but is silent about the prevention of chemical (hormonal) castration and corresponding appropriate dosage schemes. Further, WO 02/36144 is only directed to female mammals and does not disclose the treatment of male human nor does it mention overactive bladder as medicinal indication. Although WO 02/36144 mentions the theoretical use of GnRH antagonists, all examples and experimental embodiments refer to GnRH agonists used in discrete doses only: leuprolide acetate, deslorelin, triptorelin acetate, buserelin acetate in dose ranges from 3,75 mg to 12 mg. However, it is well-known to the person skilled in the art that the use of GnRH agonists in these doses inevitably leads to chemical (hormonal) castration and undesired unfavourable hormone withdrawal symptoms. Further, no experimental data are presented: the described experimental use of the GnRH agonist analogues is a mere hypothesis.

Reichler et al. describe the effect of GnRH analogues on urinary incontinence after ablation of the ovaries in dogs. Depot formulations of GnRH agonist analogues leuprolide, deslorelin, buserelin and triptorelin were used with dose ranges from 3,75 mg to 11,25 mg. Castration, i.e. reduction of gonadotrophins FSH and LH to basal or undetectable levels, is reported. The authors are silent about the use of LHRH antagonists, the prevention of chemical (hormonal) castration, corresponding appropriate dosage schemes and overactive bladder as medicinal indication (Reichler IM et al., Theriogenology 2003, 60: 1207-1216).

Nitti reviews the status quo on botulinum toxin for the treatment of idiopathic and neurogenic overactive bladder and detrusor overactivity. The use of LHRH antagonists is not mentioned (Nitti VW, Reviews in Urology 2006, 8(4): 198-208).

Reichler and co-workers studied the effect of a long acting GnRH analogue or placebo on plasma LH/FSH, urethral pressure profiles and clinical signs of urinary incontinence due to sphincter mechanism incompetence in bitches. Use of GnRH agonist analogue Leuprolide acetate in 20 or 30 mg doses is reported. It is further reported that there is a wide range in the response to the GnRH agonist analogue therapy, from ineffective to long lasting, which may be due to different responses between various GnRH analogues. It is even postulated that gonadotropins FSH and LH are unlikely involved in the pathophysiology of urinary incontinence in bitches since FSH and LH levels are no different in both successfully and unsuccessfully treated dogs. The authors are silent about the use of LHRH antagonists, the prevention of chemical (hormonal) castration, corresponding appropriate dosage schemes and overactive bladder as medicinal indication (Reichler IM et al., Theriogenology 2006, 66: 1227-1236).

Reichler and co-workers examined urodynamic parameters and plasma LH/FSH in spayed Beagle bitches before and 8 weeks after GnRH agonist analogue depot treatment. GnRH analogue leuprolide in a 30 mg 4-months depot formulation was applied. The bitches have already been castrated before the treatment, gonadotropins FSH and LH were shown to be decreased to undetectable levels. The authors are silent about the use of LHRH antagonists, the prevention of chemical (hormonal) castration, corresponding appropriate dosage schemes and overactive bladder as medicinal indication (Reichler IM et al., Theriogenology 2006, 66: 2127-2136).

Kaplan et al. demonstrate efficacy of tolterodine and tamsulosin for treatment of men with lower urinary tract symptoms and overactive bladder in a randomized controlled trial (Kaplan SA et al., JAMA 2006, 296(19): 2319-2328). The use of LHRH antagonists is not mentioned.

WO 2006/129162 is directed to anti-LHRH vaccines for the control or treatment of urinary incontinence. The use of LHRH antagonists is not mentioned.

### Description of the invention

The present invention has the object to provide novel treatments for lower urinary tract symptoms, in particular overactive bladder and/or detrusor overactivity, by which negative hormone withdrawal symptoms are prevented.

The object of the invention has surprisingly been solved in one aspect by providing at least one LHRH antagonist that can be used for the preparation of a medicament for the treatment or prophylaxis of at least one lower urinary tract symptom in mammals, wherein the at least one lower urinary tract symptom is selected from the group consisting of: "urinary incontinence, urge incontinence, overactive bladder, idiopathic overactive bladder, neurogenic overactive bladder, detrusor overactivity, idiopathic detrusor overactivity, neurogenic detrusor overactivity" and wherein the at least one LHRH antagonist is to be administered in an intermediate dose, which does not cause chemical (hormonal) castration.

The term "overactive bladder" is defined by the International Continence Society (ICS) as follows: Overactive bladder (OAB) is a symptom complex consisting of urgency with or without urge incontinence, usually with frequency and nocturia, in the absence of local pathologic or hormonal factors (Abrams P et al., Urology 2003, 61(1): 37-49; Abrams P et al., Urology 2003, 62(Supplement 5B): 28-37 and 40-42). Synonyms of overactive bladder (OAB) include "urge syndrome" and "urge frequency syndrome".

The term "detrusor overactivity" is defined by the International Continence Society (ICS) as follows: Detrusor overactivity is a urodynamic observation characterized by involuntary detrusor contractions during the filling phase that may be spontaneous or provoked (Abrams P et al., Urology 2003, 62(Supplement 5B): 28-37 and 40-42).

An excellent overview about the lower urinary tract symptoms "overactive bladder" and "detrusor overactivity" including their definitions, different subforms and/or different etiologies, in particular in men and women, is given in Abrams P et al. (Abrams P et al., Urology 2003, 62(Supplement 5B): 28-37 and 40-42). From the document it is evident that patients suffering from overactive bladder almost all have coexisting daytime and nighttime frequency and many of them also urgency, whereas only up to one half of the patients have co-existing urge incontinence (figure 1). In woman there is also a form of "dry" overactive bladder", where patients show urgency, increased frequency and nocturia, but not urge incontinence (figure 2). From this overview it is clear that "overactive bladder" and/or "detrusor overactivity" (figure 3) are not equal to urge incontinence, as many of the patients suffering from the former two syndromes are not incontinent, i.e. they are "dry" (see also: Tiwari A et al., Expert Opin. Investig. Drugs 2006, 15(9): 1017-1037). This document (Abrams P et al., Urology 2003, 62(Supplement 5B): 28-37 and 40-42) is herewith explicitly incorporated by reference in its entirety and explicitly made reference to with regard to the definitions, different subforms and/or etiologies given, in particular with respect to men and women, separately.

It is therefore preferred in the course of the present invention that all these different subforms and etiologies of "overactive bladder" and/or "detrusor overactivity" are comprised. In particular it is preferred that patients suffering from "overactive bladder" and/or "detrusor overactivity" are comprised that are not incontinent, i.e. who do not show symptoms of urge incontinence, incontinence and the like, but are "dry".

The term "intermediate dose" in the course of the present invention is defined by its higher and lower limit and has the following meaning: The higher limit of "intermediate dose" is the dose that just does not cause chemical (hormonal) castration as defined herein, wherein the lower limit of "intermediate dose" is the dose that just causes a lowering, even if a very small one, of LH, FSH and/or testosterone with regard to normal sex hormone blood levels. It lies within the knowledge of the skilled artisan to elaborate the lower and upper limit of an "intermediate dose" for each LHRH antagonist to be used on the basis of his expert knowledge and the disclosure of the present invention.

In a preferred embodiment, at least one LHRH antagonist is provided that can be used for the preparation of a medicament for the treatment or prophylaxis of at least one lower urinary tract symptom in mammals, wherein the at least one lower urinary tract symptom is selected from the group consisting of: "overactive bladder, idiopathic overactive bladder, neurogenic overactive bladder, detrusor overactivity, idiopathic detrusor overactivity, neurogenic detrusor overactivity" and wherein the at least one LHRH antagonist is to be administered in an intermediate dose, which does not cause chemical (hormonal) castration.

In another preferred embodiment, at least one LHRH antagonist is provided that can be used for the preparation of a medicament for the simultaneous treatment or prophylaxis of at least one lower urinary tract symptom selected from the group consisting of: "overactive bladder, idiopathic overactive bladder, neurogenic overactive bladder, detrusor overactivity, idiopathic detrusor overactivity, neurogenic detrusor overactivity" and benign prostatic hyperplasia (BPH), wherein the at least one LHRH antagonist is to be administered in an intermediate dose, which does not cause chemical (hormonal) castration.

That is, for instance, by means of this preferred embodiment patients can be treated that suffer from overactivce bladder and/or its subforms/etiologies idiopathic overactive bladder and/or neurogenic overactive bladder and benign prostatic hyperplasia (BPH).

In a further preferred embodiment, at least one LHRH antagonist is provided that can be used for the preparation of a medicament for the herein disclosed treatments, wherein the LHRH antagonist is selected from the group consisting of: "abarelix (Chemical Abstract Services Registry Number: 183552-38-7), antide (Chemical Abstract Services Registry Number: 112568-12-4), azaline B (Chemical Abstract Services Registry Number: 134457-28-6), A-75998 (Chemical Abstract Services Registry Number: 135215-95-1), cetrorelix (Chemical Abstract Services Registry Number: 120287-85-6), degarelix (Chemical Abstract Services Registry Number: 214766-78-6), detirelix (Chemical Abstract Services Registry Number: 89662-30-6), ozarelix (D-63153) (Chemical Abstract Services Registry Number: 295350-45-7), ganirelix (Chemical Abstract Services Registry Number: 124904-93-4), Nal-Glu antagonist, ramorelix (Chemical Abstract Services Registry Number: 127932-90-5), RS-68439 (Chemical Abstract Services Registry Number: 102583-46-0), teverelix (Chemical Abstract Services Registry Number: 144743-92-0)" and preferably is cetrorelix or ozarelix (D-63153).

In yet another preferred embodiment, at least one LHRH antagonist as defined herein is provided that can be used for the preparation of a medicament for the herein disclosed treatments, wherein the intermediate dose is a total monthly dose in the range of 10 mg to 150 mg LHRH antagonist, preferably a total monthly dose in the range of 10 mg to 120 mg LHRH antagonist, more preferably a total monthly dose in the range of 10 mg to 40 mg LHRH antagonists, more preferably a total monthly dose in the range of 40 mg to 150 mg LHRH antagonists, more preferably a total monthly dose in the range of 60 mg to 150 mg LHRH antagonists, more preferably a total monthly dose in the range of 60 mg to 120 mg LHRH antagonists and most preferably a total monthly dose of 10 mg, 20 mg, 30 mg, 40 mg, 60 mg, 90 mg, 120 mg, 130 mg or 150 mg LHRH antagonist.

In yet a further preferred embodiment, such total monthly dose is to be administered as one single monthly administration or is to be administered twice a month (preferably biweekly), three-times a month or four-times a month (preferably weekly).

If a total monthly dose is to be administered biweekly or weekly, for instance, the total monthly dose is the sum of each single administration, where the single administrations need not to be identical. That is a total monthly dose of 40 mg LHRH antagonist can, for instance, be administered in two biweekly administrations of 20 mg + 20 mg or 30 mg + 10 mg or in four weekly administrations of 10 mg + 10 mg + 10 mg + 10 mg or 20 mg + 5 mg + 10 mg + 5 mg. A total monthly dose of 90 mg LHRH antagonist can, for instance, be administered in two biweekly administrations of 60 mg + 30 mg or 30 mg + 60 mg or 45 mg + 45 mg or three-times a month as 30 mg + 30 mg + 30 mg.

In a preferred embodiment, at least one LHRH antagonist as defined herein is provided that can be used for the preparation of a medicament for the herein disclosed treatments with the herein disclosed doses, wherein the at least one LHRH antagonist is to be administered over a treatment period of 1 or 2 months followed by a treatment-free period of 1, 2, 3, 4 or 5 months, preferably 2 months or 5 months (one treatment cycle).

The term "treatment cycle" in the course of the present invention is defined as a treatment period of 1 or 2 months followed by a treatment-free period of at least one and up to five months. That is the shortest treatment cycle consists of a one-month treatment period and a one-month treatment-free period, whereas the longest treatment cycle consists of a two-months treatment period and a five-months treatment-free period. Preferred are a treatment cycle with a one-month or two-months treatment period and a two-months treatment-free period and a treatment cycle with a one-month or two-months treatment period and a five-months treatment-free period.

In a further preferred embodiment, at least one LHRH antagonist as defined herein is provided that can be used for the preparation of a medicament for the herein disclosed treatments with the herein disclosed doses, wherein the at least one LHRH antagonist is to be administered in a dose of:
- 5 mg LHRH antagonist four-times a month (preferably weekly) or three-times a month or twice a month (preferably biweekly), or
- 10 mg LHRH antagonist four-times a month (preferably weekly) or three-times a month or twice a month (preferably biweekly), or
- 15 mg LHRH antagonist four-times a month (preferably weekly) or three-times a month or twice a month (preferably biweekly), or
- 30 mg LHRH antagonist four-times a month (preferably weekly) or three-times a month or twice a month (preferably biweekly), or
- 60 mg LHRH antagonist as one single administration followed by 30 mg LHRH antagonist as one single administration two weeks later, or
- 60 mg LHRH antagonist twice a month (preferably biweekly),
over a treatment period of 1 or 2 months followed by a treatment-free period of 1, 2, 3, 4 or 5 months, preferably 2 months or 5 months (one treatment cycle).

In a yet further preferred embodiment, at least one LHRH antagonist as defined herein is provided that can be used for the preparation of a medicament for the herein disclosed treatments with the herein disclosed doses, wherein the treatment cycle is repeated after the end of the treatment-free period of the preceding treatment cycle once, twice, three-times, four-times, five-times or continuously (chronic treatment) and wherein each respective succeeding treatment cycle can be identical or different to each respective preceding treatment cycle.

That is, for instance, a treatment cycle with a one-month or two-months treatment period and a two-months treatment-free period can be followed by a treatment cycle with a one-month or two-months treatment period and a five-months treatment-free period or vice versa. A chronic treatment could, for instance, consist of consecutive treatment cycles with a one-month or two-months treatment period and a two-months treatment-free period or of consecutive treatment cycles with a one-month of two-months treatment period and a five-months treatment-free period or of consecutive treatment cycles with alternating one-month or two-months treatment periods and two-or five-months treatment-free periods in all possible ways.

It is known in the prior art, that testosterone castration levels of castrates and boys before reaching puberty are in the range between 0,3 to 1,2 ng/mL ("Labor und Diagnose, Herausgegeben von Lothar Thomas, 5. Erweiterte Auflage 2000, page 44, 44.2.5 Referenzbereich").

In the course of the present invention, the terms "hormone" and "hormonal" within for instance the terms "hormone castration", "chemical (hormonal) castration" or "hormone withdrawal symptoms" refer to follicle stimulating hormone (FSH), luteinizing hormone (LH) and/or testosterone. Preferably, chemical (hormonal) castration is a testosterone castration and refers to a testosterone blood level of equal or below 1,2 ng/mL, preferably 0,5 ng/mL.

In a preferred embodiment, at least one LHRH antagonist as defined herein is provided that can be used for the preparation of a medicament for the herein disclosed treatments with the herein disclosed doses, wherein the chemical (hormonal) castration is a testosterone castration referring to a testosterone blood level of equal or below 1,2 ng/mL, preferably 0,5 ng/mL.

In a further aspect of the present invention, the at least one LHRH antagonist as defined herein is provided that can be used for the preparation of a medicament for the herein disclosed treatments with the herein disclosed doses, wherein such medicament comprises at least one additional pharmacologically active substance.

In a yet further aspect of the present invention, the at least one LHRH antagonist as defined herein is provided that can be used for the preparation of a medicament for the herein disclosed treatments with the herein disclosed doses, wherein the medicament is applied before and/or during and/or after treatment with at least one additional pharmacologically active substance.

The above mentioned at least one additional pharmacologically active substance includes: "smooth muscle relaxants, bladder relaxants, bladder smooth muscle relaxants, anticholinergic agents, muscarinic acetylcholine receptor antagonists, tricyclic antidepressants, Calcium antagonist, Calcium agonist, Calcium channel blockers, Calcium channel activators, Potassium antagonists, Potassium agonists, Potassium channel blockers, Potassium channel activators, alpha-adrenergic receptor antagonists, alpha-blockers, alpha-adrenoreceptor antagonists, ß3-adrenoreceptor agonists, vanilloids, vanilloid receptor antagonists, botulinum toxins" and preferably, the at least one additional pharmacologically active substance is selected of the group consisting of this agents.

Examples of suitable anticholinergic agents and/or muscarinic acetylcholine receptor antagonists include oxybutynin (Benzeneacetic acid alpha-cyclohexyl-alpha-hydroxy-, 4-(diethylamino)-2-butynyl ester; synonyms: Ditropan; Ditropan XL; Oxytrol; Uromax, Chemical Abstract Services Registry Number: 5633-20-5), flavoxate (4H-1-Benzopyran-8-carboxylic acid 3-methyl-4-oxo-2-phenyl- 2-(1-piperidinyl)ethyl ester, Chemical Abstract Services Registry Number: 15301-69-6), propantheline (N-methyl-N-(1-methylethyl)-N-[2-[(9H-xanthen-9-ylcarbonyl)oxy]ethyl]-2-propanaminium, Chemical Abstract Services Registry Number: 298-50-0), dicyclomine ([1,1'-Bicyclohexyl]-1-carboxylic acid 2-(diethylamino)ethyl ester, synonyms: Bentyl; Bentylol; Dicycloverin; Dicycloverine; Diocyl; Wyovin, Chemical Abstract Services Registry Number: 77-19-0), tolterodine (2-[(1R)-3-[bis(1-methylethyl)amino]-1-phenylpropyl]-4-methyl-phenol, synonyms: Kabi 2234; PNU 200583; Chemical Abstract Services Registry Number: 124937-51-5), darifenancin (1-[2-(2,3-dihydro-5-benzofuranyl)ethyl]-alpha, alpha-diphenyl-(3S)-3-pyrrolidineacetamide, synonyms: UK 88525, Chemical Abstract Services Registry Number: 133099-04-4), solifenancin ((1S)-(3R)-1-azabicyclo[2.2.2]oct-3-yl 3,4-dihydro-1-phenyl-2(1H)-isoquinolinecarboxylate (1:1) Butanedioic acid, synonyms: Vesicare; YM 905, Chemical Abstract Services Registry Number: 242478-38-2), trospium chloride (3-[(2-hydroxy-2,2-diphenylacetyl)oxy]-, chloride (1:1), (1-alpha,3-beta,5-alpha)- Spiro[8-azoniabicyclo[3.2.1]octane-8,1'-pyrrolidinium], synonyms: Keptan; Relaspium; Sanctura; Spasmex; Spasmo 3; Spasmo-lyt; Chemical Abstract Services Registry Number: 10405-02-4), fesoterodine (Propanoic acid 2-methyl-, 2-[(1R)-3-[bis(1-methylethyl)amino]-1-phenylpropyl]-4-(hydroxymethyl)phenyl ester, Chemical Abstract Services Registry Number: 286930-02-7), imidafenacin (2-methyl-alpha, alpha-diphenyl-1H-Imidazole-1-butanamide, synonyms: KRP 197; ONO 8025, Chemical Abstract Services Registry Number: 170105-16-5), PSD-506.

Examples of suitable tricyclic antidepressants include imipramine (10,11-dihydro-N,N-dimethyl-5H-Dibenz[b,f]azepine-5-propanamine, synonyms: Antideprin; Berkomine; Melipramine, NSC 169866; Org 2463; Prazepine, Chemical Abstract Services Registry Number: 50-49-7).

Examples of suitable calcium antagonists include terodiline (N-(1,1-dimethylethyl)-alpha-methyl-gamma-phenyl-benzenepropanamine, Chemical Abstract Services Registry Number: 15793-40-5).

Examples of suitable alpha-adrenergic receptor antagonists and/or alpha-blocker and/or alpha-adrenoreceptor antagonists include terazosin (1-(4-amino-6,7-dimethoxy-2-quinazolinyl)-4-[(tetrahydro-2-furanyl)carbonyl]-piperazine, Chemical Abstract Services Registry Number: 63590-64-7), phenoxybenzamine (N-(2-chloroethyl)-N-(1-methyl-2-phenoxyethyl)-benzenemethanamine, synonyms: 688A; Bensylyt; Benzylyt; Dibenylin; Dibenyline; Dibenzyline; Phenoxybenzamine, Chemical Abstract Services Registry Number: 59-96-1), prazosin (1-(4-amino-6,7-dimethoxy-2-quinazolinyl)-4-(2-furanylcarbonyl)-Piperazine, synonyms: Furazosin; Lentopres, Chemical Abstract Services Registry Number: 19216-56-9), tamsulosin (5-[(2R)-2-[[2-(2-ethoxyphenoxy)ethyl]amino]propyl]-2-methoxy-benzenesulfonamide, synonyms: Amsulosin, Chemical Abstract Services Registry Number: 106133-20-4).

Examples of suitable β3-adrenoreceptor agonists include ritobegron ([4-[2-[[(1S,2R)-2-hydroxy-2-(4-hydroxyphenyl)-1-methylethyl]amino]ethyl]-2,5-dimethylphenoxy]-Acetic acid, Chemical Abstract Services Registry Number: 255734-04-4), YM-178, solabegron (3'-[[2-[[(2R)-2-(3-chlorophenyl)-2-hydroxyethyl]amino]ethyl]amino]-1,1'-Biphenyl]-3-carboxylic acid, Chemical Abstract Services Registry Number: 252920-94-8).

Examples of suitable vanilloids and/or vanilloid receptor antagonists include resiniferatoxin (Benzeneacetic acid, 4-hydroxy-3-methoxy-, [(2S,3aR,3bS,6aR,9aR,9bR,10R,11aR)-3a,3b,6,6a,9a,10,11,11a-octahydro-6a-hydroxy-8,10-dimethyl-11a-(1-methylethenyl)-7-oxo-2-(phenylmethyl)-7H-2,9b-epoxyazuleno[5,4-e]-1,3-benzodioxol-5-yl]methyl ester, synonyms: Daphnetoxin, Chemical Abstract Services Registry Number: 57444-62-9).

Examples of suitable botulinum toxins include botulinum toxin A (BOTOX) (synonyms: AGN 191622; Allergan; Allergan (toxin); Botox; Botox Cosmetic; Botulin neurotoxin A; Botulin toxin A; Botulinium toxin type A; Botulinum neurotoxin A; Botulinum toxin type A; CBTX-A; Dysport; Linurase; NT 201; NT 201 (toxin); Nc 224; Nc 270; Neuronox; Oculinum; Reloxin; Vistabel; Xeomin, Chemical Abstract Services Registry Number: 93384-43-1).

In a preferred embodiment, the at least one additional pharmacologically active substance is selected from the group consisting of: "smooth muscle relaxants, bladder relaxants, bladder smooth muscle relaxants, anticholinergic agents, muscarinic acetylcholine receptor antagonists, tricyclic antidepressants, Calcium antagonist, Calcium agonist, Calcium channel blockers, Calcium channel activators, Potassium antagonists, Potassium agonists, Potassium channel blockers, Potassium channel activators, alpha-adrenergic receptor antagonists, alpha-blockers, alpha-adrenoreceptor antagonists, β3-adrenoreceptor agonists, vanilloids, vanilloid receptor antagonists, botulinum toxins".

In another preferred embodiment, the at least one additional pharmacologically active substance is selected from the group consisting of: "oxybutynin, flavoxate, propantheline, dicyclomine, tolterodine, darifenancin, solifenancin, trospium chloride, fesoterodine, imidafenacin, PSD-506, imipramine, terodiline, terazosin, phenoxybenzamine, prazosin, tamsulosin, ritobegron, YM-178, solabegron, resiniferatoxin, botulinum toxin A (BOTOX)".

The at least one LHRH antagonist as defined herein and, where applicable, the at least one additional pharmacologically active substance as defined herein, can be administered to various mammalian species, including human, for the herewith disclosed treatments of such mammals.

For the purpose of the present invention, all mammalian species are regarded as being comprised. Preferably, such mammals are selected from the group consisting of "human, domestic animals, cattle, livestock, pets, cow, sheep, pig, goat, horse, pony, donkey, hinny, mule, hare, rabbit, cat, dog, guinea pig, hamster, rat, mouse". More preferably, such mammals are human, including male human and female human. Most preferably, such mammals are male human.

The treatments of the present invention are surprisingly characterized in that the people treated do not show hormone withdrawal symptoms. It was surprisingly found that the applied doses of LHRH antagonists - in the course of the favorable dosage/administration schemes - are sufficiently low to prevent chemical (hormonal) castration, in particular testosterone castration, i.e. without effecting the undesired castration side effects (hormone withdrawal symptoms), while still achieving the desired therapeutic effects, such as significant improvement of nocturia and frequency of emptying in the course of the treatment of overactive bladder and/or detrusor overactivity as well as their different subforms and/or etiologies.

What is more, combined treatment of patients suffering from overactive bladder and/or detrusor overactivity as well as benign prostatic hyperplasia (BPH) is possible with the favorable LHRH antagonist dosage/administration schemes of the present invention.

Further, with the treatments of the present invention, prevention of other side effects, such as anticholinergic side effects, such as dry mouth, urinary retention and constipation for instance, is possible.

LHRH antagonists can be prepared for use according to the present invention as illustrated in the relevant prior art. In this connection, LHRH antagonists can be present in fast-release or slow-release (depot) formulations. Slow-release (depot) formulations are preferred in order to ensure a patient-friendly treatment scheme.

Cetrorelix, for instance, can be administered in its acetate salt form, as a reconstitute of a lyophilisate (see EP 0 611 572 for preparation and process). Alternatively and preferred, it can also be applied as a slightly soluble pamoate microparticle formulation (WO 95/15767), pamoate salt (WO 02/14347) or pamoate suspension (WO 2006/069641), the latter being most preferred.

Ozarelix, for instance, can be prepared and administered as disclosed in WO 00/55190 and WO 2004/030650.

With regard to the at least one additional pharmacologically active substance, all stereoisomers are contemplated, either in a mixture or in pure or substantially pure form. The at least one additional pharmacologically active substance can have asymmetric centers at any of the carbon atoms including any one of the R radicals. Consequently, the at least one additional pharmacologically active substance can exist in the form of their racemates, in the form of the pure enantiomers and/or diastereomers or in the form of mixtures of these enantiomers and/or diastereomers. The mixtures may have any desired mixing ratio of the stereoisomers. All these different stereochemical forms and mixtures are within the scope of the present invention.

Thus, for example, the at least one additional pharmacologically active substance which has one or more centers of chirality and which occurs as racemates or as diastereomer mixtures can be fractionated by methods known per se into their optical pure isomers, i.e. enantiomers or diastereomers. The separation of the at least one additional pharmacologically active substance can take place by column separation on chiral or nonchiral phases or by recrystallization from an optionally optically active solvent or with use of an optically active acid or base or by derivatization with an optically active reagent such as, for example, an optically active alcohol, and subsequent elimination of the radical.

The at least one additional pharmacologically active substance may be present in the form of their double bond isomers as "pure" E or Z isomers, or in the form of mixtures of these double bond isomers.

Where possible, the at least one additional pharmacologically active substance may be in the form of the tautomers.

It is likewise possible for the at least one additional pharmacologically active substance to be in the form of any desired prodrugs such as, for example, esters, carbonates, carbamates, ureas, amides or phosphates, in which cases the actually biologically active form is released only through metabolism. Any compound that can be converted in vivo to provide the bioactive agent (i.e. compounds of the invention) is a prodrug within the scope and spirit of the invention.

Various forms of prodrugs are well known in the art and are described for instance in:
(i) Wermuth CG et al., Chapter 31: 671-696, The Practice of Medicinal Chemistry, Academic Press 1996;
(ii) Bundgaard H, Design of Prodrugs, Elsevier 1985; and
(iii) Bundgaard H, Chapter 5: 131-191, A Textbook of Drug Design and Development, Harwood Academic Publishers 1991.

Said references are incorporated herein by reference.

It is further known that chemical substances are converted in the body into metabolites which may where appropriate likewise elicit the desired biological effect - in some circumstances even in more pronounced form.

Any biologically active compound that was converted in vivo by metabolism from any of the at least one additional pharmacologically active substance is a metabolite within the scope and spirit of the invention.

The at least one additional pharmacologically active substance can be administered in a known manner. The route of administration may thereby be any route which effectively transports the active compound to the appropriate or desired site of action, for example orally or non-orally, in particular topically, transdermally, pulmonary, rectally, intravaginally, nasally or parenteral or by implantation. Oral administration is preferred.

The at least one additional pharmacologically active substances are converted into a form which can be administered and are mixed where appropriate with pharmaceutically acceptable carriers or diluents. Suitable excipients and carriers are described for example in Zanowiak P, Ullmann's Encyclopedia of Industrial Chemistry 2005, Pharmaceutical Dosage Forms, 1-33; Spiegel AJ et al., Journal of Pharmaceutical Sciences 1963, 52: 917-927; Czetsch-Lindenwald H, Pharm. Ind. 1961, 2: 72-74; Fiedler HP, Lexikon der Hilfsstoffe für Pharmazie, Kosmetik and angrenzende Gebiete 2002, Editio Cantor Verlag, p65-68.

Oral administration can take place for example in solid form as tablet, capsule, gel capsule, coated tablet, granulation or powder, but also in the form of a drinkable solution. The at least one additional pharmacologically active substance can for oral administration be combined with known and ordinarily used, physiologically tolerated excipients and carriers such as, for example, gum arabic, talc, starch, sugars such as, for example, mannitol, methylcellulose, lactose, gelatin, surface-active agents, magnesium stearate, cyclodextrins, aqueous or nonaqueous carriers, diluents, dispersants, emulsifiers, lubricants, preservatives and flavorings (e.g. essential oils). The at least one additional pharmacologically active substance can also be dispersed in a microparticulate, e.g. nanoparticulate, composition.

Non-oral administration can take place for example by intravenous, subcutaneous, intramuscular injection of sterile aqueous or oily solutions, suspensions or emulsions, by means of implants or by ointments, creams or suppositories. Administration as sustained release form is also possible where appropriate. Implants may comprise inert materials, e.g. biodegradable polymers or synthetic silicones such as, for example, silicone rubber. Intravaginal administration is possible for example by means of vaginal rings. Intrauterine administration is possible for example by means of diaphragms or other suitable intrauterine devices. Transdermal administration is additionally provided, in particular by means of a formulation suitable for this purpose and/or suitable means such as, for example, patches.

The dosage may vary within a wide range depending on type and/or severity of the physiological and/or pathophysiological condition, the mode of administration, the age, gender, bodyweight and sensitivity of the subject to be treated. It is within the ability of a skilled worker to determine a "pharmacologically effective amount" of an LHRH antagonist of the invention and/or additional pharmacologically active substance. Administration can take place in a single dose or a plurality of separate dosages.

A suitable unit dose is, for example for the at least one additional pharmacologically active substance, from 0.001 mg to 100 mg of the active ingredient, i.e. at least one additional pharmacologically active substance, per kg of a patient's bodyweight.

The contents of all cited references are hereby incorporated by reference in their entirety. The invention is explained in more detail by means of the following examples without, however, being restricted thereto.

### Examples

### Example 1:

Study subjects were treated with Cetrorelix pamoate with the following doses according to the following administration schemes:
- 60 mg Cetrorelix pamaote administered at week 0 and 30 mg Cetrorelix pamaote administered at week 2 (treatment period) followed by up to 5 months of no treatment (treatment-free period)
- 60 mg Cetrorelix pamaote administered at week 0 and 60 mg Cetrorelix pamaote administered at week 2 (treatment period) followed by up to 5 months of no treatment (treatment-free period)

The results for the placebo-controlled treatments with 60+30 mg Cetrorelix pamoate and 60+60 mg Cetrorelix pamoate are illustrated below.

Table 1 shows the results for nocturia, i.e. how often the study subjects got up during the night in order to urinate. The results shown are percentage variations from baseline at the beginning of the study (week 0).

**Table 1: Cetrorelix pamoate - Nocturia (percentage variation from baseline)**

| **Group** | **Placebo** | **60+30** | **60+60** |
|---|---|---|---|
| **Week 0** | 0 | 0 | 0 |
| **Week 4** | -17 | -14 | -17 |
| **Week 8** | -15 | -26 | -26 |
| **Week 12** | -23 | -27 | -30 |
| **Week 16** | -23 | -27 | -25 |
| **Week 20** | -24 | -33 | -27 |
| **Week 24** | -23 | -26 | -29 |

The results indicate that there is a placebo effect. However, treatments with Cetrorelix pamoate according to above doses and administration schemes demonstrate the effectiveness of the treatments of the invention. An **up to 11%** further decrease in nocturia frequency for the treatments of the invention could be observed compared to placebo.

Table 2 shows the results for frequency of emptying, i.e. how often the study subjects had to urinate a second time within a 2 hour test period. The results shown are percentage variations from baseline at the beginning of the study (week 0).

**Table 2: Cetrorelix pamoate - Frequency of emptying (percentage variation from baseline)**

| **Group** | **Placebo** | **60+30** | **60+60** |
|---|---|---|---|
| **Week 0** | 0 | 0 | 0 |
| **Week 4** | -10 | -9 | -16 |
| **Week 8** | -8 | -19 | -25 |
| **Week 12** | -13 | -20 | -24 |
| **Week 16** | -13 | -26 | -29 |
| **Week 20** | -6 | -27 | -25 |
| **Week 24** | -5 | -31 | -34 |

Again, the results indicate that there is a placebo effect. However, treatments with Cetrorelix pamoate according to above doses and administration schemes demonstrate the effectiveness of the treatments of the invention. An **up to 29%** further decrease in frequency for the treatments of the invention could be observed compared to placebo.

Table 3 shows the results for the IPSS irritative subscore, i.e. the sum of the effects of nocturia, frequency of emptying and urgency to urinate. The results shown are percentage variations from baseline at the beginning of the study (week 0).

**Table 3: Cetrorelix pamoate - IPSS irritative subscore (nocturia, frequency of emptying, urgency to urinate)**

| **Group** | **Placebo** | **60+30** | **60+60** |
|---|---|---|---|
| **Week 0** | 0 | 0 | 0 |
| **Week 4** | -11,8 | -14,8 | -16,7 |
| **Week 8** | -15,5 | -33,3 | -25 |
| **Week 12** | -16,7 | -29,3 | -25 |
| **Week 16** | -14,3 | -33,3 | -25 |
| **Week 20** | -14,3 | -33,3 | -27,6 |
| **Week 24** | -10 | -38 | -40 |

Again, the results indicate that there is a placebo effect. However, treatments with Cetrorelix pamoate according to above doses and administration schemes demonstrate the effectiveness of the treatments of the invention. An **up to 30%** further decrease in IPSS irritative subscore for the treatments of the invention could be observed compared to placebo.

Table 4 shows the corresponding median testosterone blood levels [ng/mL] for the treatments with Cetrorelix pamoate according to above doses and administration schemes as well as for the placebo treatment.

**Table 4: Cetrorelix pamoate - Testosterone [ng/mL] (median)**

| **Group** | **Placebo** | **60+30** | **60+60** |
|---|---|---|---|
| **Week 0** | 4,23 | 3,96 | 3,71 |
| **Week 4** | 4,01 | 2,59 | 2,19 |
| **Week 8** | 4,30 | 4,01 | 3,70 |
| **Week 12** | 4,00 | 4,10 | 3,73 |
| **Week 16** | 4,09 | 3,96 | n.d. |
| **Week 20** | 3,70 | 4,12 | n.d. |
| **Week 24** | 3,67 | 4,30 | n.d. |

The results clearly demonstrate that chemical (hormonal) castration could be successfully prevented.

### Example 2:

Study subjects were treated with Cetrorelix acetate with the following doses according to the following administration schemes:
- 5 mg Cetrorelix acetate administered at week 0, 1, 2 and 3 (total monthly dose of 20 mg) (treatment period) followed by up to 4 months of no treatment (treatment-free period)
- 10 mg Cetrorelix acetate administered at week 0 and 10 mg Cetrorelix acetate administered at week 2 (treatment period) followed by up to 4 months of no treatment (treatment-free period)
- 10 mg Cetrorelix acetate administered at week 0, 1, 2 and 3 (total monthly dose of 40 mg) (treatment period) followed by up to 4 months of no treatment (treatment-free period)

The results for the placebo-controlled treatments with 5+5+5+5 mg Cetrorelix acetate, 10+10 mg Cetrorelix acetate and 10+10+10+10 mg Cetrorelix acetate are illustrated below.

Table 5 shows the results for nocturia, i.e. how often the study subjects got up during the night in order to urinate. The results shown are mean values, how often the study subjects had to get up.

**Table 5: Cetrorelix acetate - Nocturia (n-times, mean)**

| **Group** | **Placebo** | **4x5** | **2x10** | **4x10** |
|---|---|---|---|---|
| **Week 0** | 2,71 | 2,57 | 2,69 | 2,63 |
| **Week 4** | 2,57 | 2,09 | 2,00 | 1,79 |
| **Week 8** | 2,49 | 1,94 | 2,06 | 1,78 |
| **Week 12** | 2,43 | 1,85 | 1,97 | 1,75 |
| **Week 16** | 2,46 | 1,79 | 1,97 | 1,87 |
| **Week 20** | 2,65 | 1,85 | 2,16 | 2,16 |

The results indicate that there is a slight placebo effect. However, treatments with Cetrorelix acetate according to above doses and administration schemes demonstrate the effectiveness of the treatments of the invention. An **up to 0,71** decrease in nocturia frequency for the treatments of the invention could be observed compared to placebo.

Table 6 shows the results for frequency of emptying, i.e. how often the study subjects had to urinate a second time within a 2 hour test period. The results shown are mean values, how often the study subjects had to urinate a second time.

**Table 6: Cetrorelix acetate - Frequency of emptying (n-times, mean)**

| **Group** | **Placebo** | **4x5** | **2x10** | **4x10** |
|---|---|---|---|---|
| **Week 0** | 2,40 | 2,49 | 2,29 | 2,86 |
| **Week 4** | 2,49 | 2,23 | 1,86 | 1,62 |
| **Week 8** | 2,60 | 2,00 | 1,58 | 1,66 |
| **Week 12** | 2,71 | 1,73 | 1,55 | 1,81 |
| **Week 16** | 2,80 | 1,52 | 1,45 | 1,65 |
| **Week 20** | 2,71 | 1,79 | 1,66 | 1,78 |

The results for the treatments with Cetrorelix acetate according to above doses and administration schemes demonstrate the effectiveness of the treatments of the invention. An **up to 1,35** decrease in frequency of emptying for the treatments of the invention could be observed compared to placebo.

Table 7 shows the results for the IPSS irritative subscore, i.e. the sum of the effects of nocturia, frequency of emptying and urgency to urinate. The results shown are percentage variations from baseline at the beginning of the study (week 0).

**Table 7: Cetrorelix acetate - IPSS irritative subscore (nocturia, frequency of emptying, urgency to urinate)**

| **Group** | **Placebo** | **4x5** | **2x10** | **4x10** |
|---|---|---|---|---|
| **Week 0** | 0 | 0 | 0 | 0 |
| **Week 4** | -12,5 | -16,7 | -25 | -29,3 |
| **Week 8** | -14,3 | -25 | -33,3 | -31 |
| **Week 12** | 0 | -28,6 | -33,3 | -25 |
| **Week 16** | 0 | -30,8 | -42,9 | -28,6 |
| **Week 20** | 0 | -29,7 | -29,2 | -25 |

The results indicate that there is a slight placebo effect. However, treatments with Cetrorelix acetate according to above doses and administration schemes demonstrate the effectiveness of the treatments of the invention. An **up to 43%** further decrease in IPSS irritative subscore for the treatments of the invention could be observed compared to placebo.

Table 8 shows the corresponding median testosterone blood levels [ng/mL] for the treatments with Cetrorelix acetate according to above doses and administration schemes as well as for the placebo treatment.

**Table 8: Cetrorelix acetate - Testosterone [ng/mL] (median)**

| **Group** | **Placebo** | **4x5** | **2x10** | **4x10** |
|---|---|---|---|---|
| **Week 0** | 3,93 | 3,42 | 3,27 | 3,41 |
| **Week 4** | 3,18 | 2,95 | 4,41 | 2,17 |
| **Week 8** | 3,39 | 3,77 | 3,73 | 3,82 |
| **Week 12** | 3,06 | 4,11 | 4,45 | 4,32 |
| **Week 16** | 3,77 | 3,91 | 4,16 | 4,32 |
| **Week 20** | 4,02 | 3,49 | 4,12 | 4,06 |

The results clearly demonstrate that chemical (hormonal) castration could be successfully prevented.

### Example 3:

Study subjects were treated with Ozarelix with the following doses according to the following administration schemes:
- 15 mg Ozarelix administered at week 0 and 15 mg Ozarelix administered at week 2 (treatment period) followed by up to 4 months of no treatment (treatment-free period)

The results for the placebo-controlled treatments with 15+15 mg Ozarelix are illustrated below.

Table 9 shows the results for nocturia, i.e. how often the study subjects got up during the night in order to urinate. The results shown are mean values, how often the study subjects had to get up.

**Table 9: Ozarelix - Nocturia (n-times, mean)**

| **Group** | **Placebo** | **15+15** |
|---|---|---|
| **Week 0** | 2,92 | 2,73 |
| **Week 4** | 2,33 | 1,83 |
| **Week 8** | 2,04 | 1,43 |
| **Week 12** | 2,17 | 1,54 |
| **Week 16** | 2,17 | 1,54 |
| **Week 20** | 2,26 | 1,50 |
| **Week 24** | 2,23 | 1,54 |

The results indicate that there is a placebo effect. However, treatments with Ozarelix according to above doses and administration schemes demonstrate the effectiveness of the treatments of the invention. An **up to 0,76** decrease in nocturia frequency for the treatments of the invention could be observed compared to placebo.

Table 10 shows the results for frequency of emptying, i.e. how often the study subjects had to urinate a second time within a 2 hour test period. The results shown are mean values, how often the study subjects had to urinate a second time.

**Table 10: Ozarelix - Frequency of emptying (n-times, mean)**

| **Group** | **Placebo** | **15+15** |
|---|---|---|
| **Week 0** | 2,75 | 2,90 |
| **Week 4** | 2,46 | 2,48 |
| **Week 8** | 2,00 | 2,11 |
| **Week 12** | 2,09 | 1,82 |
| **Week 16** | 2,04 | 1,75 |
| **Week 20** | 2,00 | 1,71 |
| **Week 24** | 2,05 | 1,75 |

Again, the results indicate that there is a placebo effect. However, the treatments with Ozarelix according to above doses and administration schemes demonstrate the effectiveness of the treatments of the invention. An **up to 0,30** decrease in frequency of emptying for the treatments of the invention could be observed compared to placebo.

Table 11 shows the results for urgency to urinate, i.e. how often the study subjects had difficulties to delay the urination. The results shown are mean values, how often the study subjects had difficulties.

**Table 11: Ozarelix - Urgency to urinate (n-times, mean)**

| **Group** | **Placebo** | **15+15** |
|---|---|---|
| **Week 0** | 2,92 | 2,80 |
| **Week 4** | 2,04 | 2,17 |
| **Week 8** | 2,13 | 1,89 |
| **Week 12** | 2,22 | 1,75 |
| **Week 16** | 2,13 | 1,39 |
| **Week 20** | 2,26 | 1,71 |
| **Week 24** | 2,09 | 1,89 |

Again, the results indicate that there is a placebo effect. However, the treatments with Ozarelix according to above doses and administration schemes demonstrate the effectiveness of the treatments of the invention. An **up to 0,74** decrease in urgency to urinate for the treatments of the invention could be observed compared to placebo.

Table 12 shows the corresponding median testosterone blood levels [ng/mL] for the treatments with Ozarelix according to above doses and administration schemes as well as for the placebo treatment.

**Table 12: Ozarelix - Testosterone [ng/mL] (median)**

| **Group** | **Placebo** | **15+15** |
|---|---|---|
| **Week 0** | 3,95 | 3,57 |
| **Week 4** | 4,16 | 2,97 |
| **Week 8** | 3,99 | 4,19 |
| **Week 12** | 3,98 | 4,11 |
| **Week 16** | 4,00 | 3,82 |
| **Week 20** | 3,50 | 3,92 |
| **Week 24** | 4,22 | 3,86 |

The results clearly demonstrate that chemical (hormonal) castration could be successfully prevented.

## Claims

1. Use of at least one LHRH antagonist for the preparation of a medicament for the treatment or prophylaxis of at least one lower urinary tract symptom in mammals, wherein the at least one lower urinary tract symptom is selected from the group consisting of: "urinary incontinence, urge incontinence, overactive bladder, idiopathic overactive bladder, neurogenic overactive bladder, detrusor overactivity, idiopathic detrusor overactivity, neurogenic detrusor overactivity" and wherein the at least one LHRH antagonist is to be administered in an intermediate dose, which does not cause chemical (hormonal) castration.

2. The use as claimed in claim 1, wherein the at least one lower urinary tract symptom is selected from the group consisting of: "overactive bladder, idiopathic overactive bladder, neurogenic overactive bladder, detrusor overactivity, idiopathic detrusor overactivity, neurogenic detrusor overactivity".

3. The use as claimed in claim 1 for the preparation of a medicament for the simultaneous treatment or prophylaxis of at least one lower urinary tract symptom selected from the group consisting of: "overactive bladder, idiopathic overactive bladder, neurogenic overactive bladder, detrusor overactivity, idiopathic detrusor overactivity, neurogenic detrusor overactivity" and benign prostatic hyperplasia (BPH).

4. The use as claimed in any of claims 1 to 3, wherein the LHRH antagonist is selected from the group consisting of: "abarelix, antide, azaline B, A-75998, cetrorelix, degarelix, detirelix, ozarelix (D-63153), ganirelix, Nal-Glu-Antagonist, ramorelix, RS-68439, teverelix" and preferably is cetrorelix or ozarelix (D-63153).

5. The use as claimed in any of claims 1 to 4, wherein the intermediate dose is a total monthly dose in the range of 10 mg to 150 mg LHRH antagonist, preferably a total monthly dose in the range of 10 mg to 120 mg LHRH antagonist, more preferably a total monthly dose in the range of 10 mg to 40 mg LHRH antagonists, more preferably a total monthly dose in the range of 40 mg to 150 mg LHRH antagonists, more preferably a total monthly dose in the range of 60 mg to 150 mg LHRH antagonists, more preferably a total monthly dose in the range of 60 mg to 120 mg LHRH antagonists and most preferably a total monthly dose of 10 mg, 20 mg, 30 mg, 40 mg, 60 mg, 90 mg, 120 mg, 130 mg or 150 mg LHRH antagonist.

6. The use as claimed in any of claims 1 to 5, wherein the total monthly dose is to be administered as one single monthly administration or is to be administered twice a month (preferably biweekly), three-times a month or four-times a month (preferably weekly).

7. The use as claimed in any of claims 1 to 6, wherein the at least one LHRH antagonist is to be administered over a treatment period of 1 or 2 months followed by a treatment-free period of 1, 2, 3, 4 or 5 months, preferably 2 months or 5 months (one treatment cycle).

8. The use as claimed in any of claims 1 to 7, wherein the at least one LHRH antagonist is to be administered in a dose of:
- 5 mg LHRH antagonist four-times a month (preferably weekly) or three-times a month or twice a month (preferably biweekly), or
- 10 mg LHRH antagonist four-times a month (preferably weekly) or three-times a month or twice a month (preferably biweekly), or
- 15 mg LHRH antagonist four-times a month (preferably weekly) or three-times a month or twice a month (preferably biweekly), or
- 30 mg LHRH antagonist four-times a month (preferably weekly) or three-times a month or twice a month (preferably biweekly), or
- 60 mg LHRH antagonist as one single administration followed by 30 mg LHRH antagonist as one single administration two weeks later, or
- 60 mg LHRH antagonist twice a month (preferably biweekly),
over a treatment period of 1 or 2 months followed by a treatment-free period of 1, 2, 3, 4 or 5 months, preferably 2 months or 5 months (one treatment cycle).

9. The use as claimed in any of claims 7 to 8, wherein the treatment cycle is repeated after the end of the treatment-free period of the preceding treatment cycle once, twice, three-times, four-times, five-times or continuously (chronic treatment) and wherein each respective succeeding treatment cycle can be identical or different to each respective preceding treatment cycle.

10. The use as claimed in any of claims 1 to 9, wherein the chemical (hormonal) castration is a testosterone castration referring to a testosterone blood level of equal or below 1,2 ng/mL, preferably 0,5 ng/mL.

11. The use as claimed in any of claims 1 to 10, where such medicament comprises at least one additional pharmacologically active substance.

12. The use as claimed in any of claims 1 to 10, where the medicament is applied before and/or during and/or after treatment with at least one additional pharmacologically active substance.

13. The use as claimed in any of claims 11 to 12, where such at least one additional pharmacologically active substance is selected from the group consisting of: "smooth muscle relaxants, bladder relaxants, bladder smooth muscle relaxants, anticholinergic agents, muscarinic acetylcholine receptor antagonists, tricyclic antidepressants, Calcium antagonist, Calcium agonist, Calcium channel blockers, Calcium channel activators, Potassium antagonists, Potassium agonists, Potassium channel blockers, Potassium channel activators, alpha-adrenergic receptor antagonists, alpha-blockers, alpha-adrenoreceptor antagonists, β3-adrenoreceptor agonists, vanilloids, vanilloid receptor antagonists, botulinum toxins".

14. The use as claimed in any of claims 11 to 13, where such at least one additional pharmacologically active substance is selected from the group consisting of: "oxybutynin, flavoxate, propantheline, dicyclomine, tolterodine, darifenancin, solifenancin, trospium chloride, fesoterodine, imidafenacin, PSD-506, imipramine, terodiline, terazosin, phenoxybenzamine, prazosin, tamsulosin, ritobegron, YM-178, solabegron, resiniferatoxin, botulinum toxin A (BOTOX)".
